(19) Europäisches Patentamt / European Patent Office / Office européen des brevets

(11) **EP 2 617 429 B1**

(12) **EUROPEAN PATENT SPECIFICATION**

(45) Date of publication and mention
of the grant of the patent:
**07.01.2015 Bulletin 2015/02**

(21) Application number: **11825208.9**

(22) Date of filing: **14.09.2011**

(51) Int Cl.:
*A61K 36/18* (2006.01)    *A61K 31/05* (2006.01)
*A61K 31/216* (2006.01)   *A61K 36/00* (2006.01)
*A61P 3/04* (2006.01)     *A61P 3/06* (2006.01)
*A23L 1/30* (2006.01)

(86) International application number:
**PCT/JP2011/071001**

(87) International publication number:
**WO 2012/036208 (22.03.2012 Gazette 2012/12)**

(54) **FAT OXIDATION OR ENERGY METABOLISM ENHANCER**

FETTVERBRENNUNGS- ODER ENERGIESTOFFWECHSELVERSTÄRKER

AGENT D'AMÉLIORATION DE L'OXYDATION DES GRAISSES ET DU MÉTABOLISME ÉNERGÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.09.2010 JP 2010207465**

(43) Date of publication of application:
**24.07.2013 Bulletin 2013/30**

(73) Proprietor: **Kao Corporation**
**Chuo-ku**
**Tokyo 103-8210 (JP)**

(72) Inventors:
• **SOGA, Satoko**
**Haga-gun, Tochigi 321-3497 (JP)**
• **OTA, Noriyasu**
**Haga-gun, Tochigi 321-3497 (JP)**
• **SHIMOTOYODOME, Akira**
**Haga-gun, Tochigi 321-3497 (JP)**
• **MURASE, Takatoshi**
**Haga-gun, Tochigi 321-3497 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 119 368**    **JP-A- 2003 034 636**
**JP-A- 2006 271 392**    **JP-A- 2008 043 238**

• **CHO A S ET AL: "Chlorogenic acid exhibits anti-obesity property and improves lipid metabolism in high-fat diet-induced-obese mice", FOOD AND CHEMICAL TOXICOLOGY, PERGAMON, GB, vol. 48, no. 3, 1 March 2010 (2010-03-01), pages 937-943, XP027583308, ISSN: 0278-6915 [retrieved on 2010-01-12]**
• **SANDER KERSTEN: 'Peroxisome proliferator activated receptors and obesity' EUR. J. PHARMACOL. vol. 440, no. 2-3, 12 April 2002, pages 223 - 234, XP055076610**
• **MOL. MED. vol. 39, no. 3, 2002, pages 302 - 311**

**Description**

[Field of the Invention]

The present invention relates to a fat oxidation or energy metabolism enhancer.

[Background of the Invention]

Obesity refers to a condition in which an individual weighs more than usual as a result of excessive accumulation of energy intake from carbohydrate, fat, and the like in the form of fat under the skin or around the viscera. Also, recently, a concept of metabolic syndrome has been gaining attention, where metabolic syndrome refers to a condition associated with underlying excessive accumulation of visceral fat (obesity), in which one is likely to develop arteriosclerosis complicated with abnormal glucose tolerance (hyperglycemia), high triglyceride (hyperlipidemia), and hypertension. Namely, metabolic syndrome refers to a condition in which one faces an increased risk of developing lifestyle disease.

In Japan, according to the diagnostic criteria for metabolic syndrome published in 2005, males having a waist circumference of 85 cm or more or females having a waist circumference of 90 cm or more who satisfy two or more items of the following three items, which are (1) having blood triglyceride of 150 mg/dl or more or HDL cholesterol of less than 40 mg/dl, (2) having hyperglycemia (fasting blood glucose of 110 mg/dl or more), and (3) having hypertension (130/85 mHg or more), are determined to have metabolic syndrome. Further, according to the investigation conducted by Ministry of Health, Labour and Welfare based on the diagnostic criteria (May, 2006), 13 million Japanese qualify as having metabolic syndrome, with the number reaching 27 million when those who are likely to develop metabolic syndrome are included. Considering that an increase in patients with obesity and metabolic syndrome may lead to an increase in medical costs, obesity and metabolic syndrome are becoming major issues around the world not just in Japan.

Obesity is induced when the amount of energy intake exceeds the amount of energy consumed. Therefore, in order to ameliorate obesity, a method of decreasing the amount of energy intake from fat, carbohydrate, and the like or a method for increasing the amount of energy consumption by enhancing in vivo metabolism by some methods are possible.

Exercise is effective for prevention and amelioration of obesity and metabolic syndrome as a method for activating in vivo metabolism and increasing energy consumption. However, exercising on a continual basis is practically difficult. Therefore, development of a substance or a method which increases energy metabolism, for example, a substance or a method which increases the amount of energy consumed in daily life without requiring special training and the like by enhancing oxidation of carbohydrate and fat, is demanded.

Chlorogenic acid (5-caffeoylquinic acid), which is one of coffee polyphenols, has been so far reported to have various physiological actions. For example, it is known that chlorogenic acid has a fatty acid synthase-inhibiting action (Non Patent Document 1), and coffee containing chlorogenic acid is known to have a blood glucose absorption-inhibiting action and a body weight-suppressing action (Non Patent Document 2). Further, it is also known that chlorogenic acids have a fat metabolism-activating action by activation of the peroxisome proliferator-activated receptor (PPAR) (Patent Document 1).

Hydroxyhydroquinone (HHQ) is a component contained in a coffee drink, and is known to have unfavorable actions such as generating hydrogen peroxide in vivo.

For example, despite the fact that chlorogenic acid has a hypotensive action, a coffee drink containing a large amount of hydroxyhydroquinone does not always bring about a sufficient hypotensive action (Non Patent Document 3). In view of this, a coffee drink composition with hydroxyhydroquinone removed has been proposed (Patent Literatures 2 and 3) .

[Citation List]

[Patent Document]

[Patent Document 1] JP-A-2003-34636
[Patent Document 2] JP-A-2006-271392
[Patent Document 3] JP-A-2008-43238

[Non Patent Document]

[Non Patent Document 1] IUBMB Life, 58 (1), 39 to 46, 2006
[Non Patent Document 2] J. Int. Med. Res. 35 (6), 900 to 908, 2007
[Non Patent Document 3] Eur. J. Clin. Nutr., 53 (11), 831, 1999

Cho et al., Food and Chemical Toxicology 48(3), 937-943 (2010) examine the effect of chlorogenic acid and caffeic acid on the fat metabolism in mice fed with a high fat diet. Inter alia, the activities of fatty acid synthase, fatty acid beta oxidation, HMG-CoA reductase and ACAT are examined. The authors conclude that that chlorogenicacid down-regulates fatty acid synthesis and promotes fatty acid oxidation.

EP 2 119 368 relates to refined roasted coffee beans having a Hunter L value of 10 to 30 and a hydroxyhydroquinone content of not higher than 30 mg/kg, which are prepared by contacting an aqueous solvent with raw material roasted coffee beans.

[Summary of the Invention]

One aspect of the present invention relates to a roasted coffee bean extract for use in a method for preventing, ameliorating or reducing the risk of developing obesity or metabolic syndrome by enhancement of postprandial fat oxidation or postprandial energy metabolism, comprising chlorogenic acids or salts thereof and having a content of hydroxyhydroquinone of less than 0.1 % by mass relative to a content of the chlorogenic acids.

Another aspect of the present invention relates to the non-therapeutic use of a roasted coffee bean extract for enhancement of postprandial fat oxidation, comprising chlorogenic acids or salts thereof and having a content of hydroxyhydroquinone of less than 0.1% by mass relative to a content of the chlorogenic acids.

A further aspect of the invention relates to the non-therapeutic use of a roasted coffee bean extract for enhancement of postprandial energy metabolism, comprising chlorogenic acids or salts thereof and having a content of hydroxyhydroquinone of less than 0.1% by mass relative to a content of the chlorogenic acids.

[Brief Description of Drawings]

[Figure 1] Figure 1 is a graph illustrating the mean postprandial values of the respiratory exchange ratio (*: t-test, p < 0.05).

[Figure 2] Figure 2 is a graph illustrating the mean postprandial values of the amount of fat oxidation (*: t-test, p < 0.05).

[Detailed Description of the Invention]

In the present specification, the term "non-therapeutic" refers to a concept which does not include medical practice, i.e., the practice of treatment of the human body by therapy.

The present invention may be used in the form of a highly safe composition, a pharmaceutical product, a quasi-drug, a cosmetic, a food, a drink, a pet food, a feed, and the like which have excellent facilitatory actions on fat oxidation and energy metabolism and are effective for, for example, reducing the risk of developing obesity or metabolic syndrome or preventing and ameliorating obesity or metabolic syndrome. The provision of a raw material for the products is also described.

The present inventors found that excellent facilitatory actions on fat oxidation and energy metabolism can be achieved by reducing the content of hydroxyhydroquinone in a roasted coffee bean extract to less than 0.1% by mass relative to the content of chlorogenic acids.

The roasted coffee bean extract containing chlorogenic acids or salts thereof and having the content of hydroxyhydroquinone of less than 0.1% by mass relative to the content of the chlorogenic acids according to the present invention has excellent facilitatory actions on fat oxidation and energy metabolism. Hence, according to the present invention, a pharmaceutical product, a quasi-drug, a cosmetic, a food, a drink, a pet food, and a feed for, for example, reducing the risk of developing obesity or metabolic syndrome or preventing and ameliorating obesity or metabolic syndrome, as well as a raw material for the products, can be provided.

A method of roasting and a method of extraction for the roasted coffee bean extract used in the present invention are not particularly limited; however, for example, a roasted coffee bean extract prepared by a method such as subjecting a coffee bean roasted over an open fire or a pulverized product thereof to extraction by boiling method can be used. Alternatively, a commercially available roasted coffee bean extract (for example, BRA NO.2 L22/26 blend beans, manufactured by Unicafe, Inc.) may also be used.

The roasted coffee bean extract used in the present invention may be obtained by subjecting the aforementioned extract to further one treatment or an appropriate combination of treatments such as concentration, dilution, and freeze-drying or spray-drying.

The roasted coffee bean extract used in the present invention may be in any form including a solid such as a powder, a granule, and a block, a semi-solid such as a gel, and a liquid.

In the present invention, examples of the chlorogenic acids include a compound in which one to two hydroxyl groups selected from the positions 3, 4, and 5 of quinic acid are ester-linked to caffeic acid and/or ferulic acid. Specific examples thereof include 3-caffeoylquinic acid, 4-caffeoylquinic acid, 5-caffeoylquinic acid (chlorogenic acid), 3,4-dicaffeoylquinic acid, 3,5-dicaffeoylquinic acid, and 4,5-dicaffeoylquinic acid, and feruloylquinic acids such as 3-feruloylquinic acid, 4-feruloylquinic acid, and 5-feruloylquinic acid.

Examples of the salt of chlorogenic acids include a salt with an alkali metal such as sodium and potassium, a salt with an alkaline earth metal such as magnesium and calcium, a salt with an organic amine such as monoethanolamine, diethanolamine, and triethanolamine, and a salt with a basic amino acid such as arginine, lysine, histidine, and ornithine. Here, the chlorogenic acids encompass ones containing free carboxyl groups.

The roasted coffee bean extract used in the present invention may contain one of these chlorogenic acids or salts thereof or a combination of two or more of them. The content is, in terms of chlorogenic acids, 2 to 20% by mass, preferably 5 to 18% by mass, more preferably 7 to 15% by mass relative to the total solid mass of the extract. The content of chlorogenic acids in the roasted coffee bean extract can be measured by high-performance liquid chromatography (HPLC). While UV detection is commonly employed as means of detection by HPLC, chlorogenic acids can also be detected with higher sensitivity by chemiluminescence (CL) detection, electrochemistry (EC) detection, LC-Mass detection, and the like.

The content of hydroxyhydroquinone in the aforementioned roasted coffee bean extract is less than 0.1% by mass, preferably 0.03% by mass or less, more preferably 0.01% by mass or less relative to the amount of chlorogenic acids. Here, the content of hydroxyhydroquinone in the extract may also be zero.

The content of hydroxyhydroquinone in the aforementioned roasted coffee bean extract can be measured by high-performance liquid chromatography (HPLC). While UV detection is commonly employed as means of detection by HPLC, hydroxyhydroquinone can also be detected with higher sensitivity by chemiluminescence (CL) detection, electrochemistry (EC) detection, LC-Mass detection, and the like. Also, while the content of hydroxyhydroquinone can be directly measured by HPLC, it can also be quantitated by concentrating hydroxyhydroquinone from the roasted coffee bean extract by any chromatography, and then measuring the amount of the concentrated fraction. The amounts of chlorogenic acids and hydroxyhydroquinone are preferably measured, for example, by adding hydrochloric acid to the extract so as to provide 0.1 N (normal) or in a 0.1 N hydrochloric acid/sodium hydroxide buffer system. It is desirable to keep the extract before measurement out of contact with oxygen.

As mentioned earlier, the content of hydroxyhydroquinone in the roasted coffee bean extract used in the present invention is reduced to less than 0.1% by mass relative to the amount of chlorogenic acids. Accordingly, the roasted coffee bean extract may be subjected to a hydroxyhydroquinone removal treatment as needed. Examples of the hydroxyhydroquinone removal treatment include a method of treating a roasted coffee bean extract with an adsorbent such as activated carbon and a reversed-phase carrier and a method of bringing a roasted coffee bean extract into contact with acid clay (JP-A-2006-087306 and JP-A-2006-117631).

As a method of treatment with an adsorbent, for example, it may be possible to add an adsorbent to a roasted coffee bean liquid extract or an aqueous solution of a dried roasted coffee bean extract, stir the resulting solution at 0 to 100°C for 10 minutes to five hours, and then remove the adsorbent. Here, when activated carbon is used as the adsorbent, it is preferably used in an amount of 0.02 to 1.0 times the weight of the solid content of the roasted coffee bean extract, while when a reversed-phase carrier is used as the adsorbent, it is preferably used in an amount of 2 to 100 times the weight of the solid content of the roasted coffee bean extract. Regarding the activated carbon, the average pore radius in the micropore region is preferably 5 angstroms (Å) or less, more preferably in the range of 2 to 5 angstroms, and even more preferably in the range of 3 to 5 angstroms. As to the kind of the activated carbon, coconut shell activated carbon is preferable, and steam-activated coconut shell activated carbon is more preferable. As a commercial product of activated carbon, Shirasagi WH2c (Japan EnviroChemicals, Ltd.), Taiko CW (Futamura Chemical Co., Ltd.), Kuraray Coal GW (Kuraray Chemical Co., Ltd.) and the like can be used. Examples of the reversed-phase carrier include YMC·ODS-A (YMC) and C18 (GL Sciences Inc.). A method of adsorbent treatment using activated carbon is preferable since it can selectively reduce the content of hydroxyhydroquinone without reducing the amount of chlorogenic acids.

As will be demonstrated in Examples to follow, compared to a roasted coffee bean extract containing a normal amount of hydroxyhydroquinone, the roasted coffee bean extract having a reduced hydroxyhydroquinone content thus obtained exerts an excellent facilitatory action on fat oxidation by inducing postprandial energy metabolism in favor of fat oxidation. In other words, compared to a roasted coffee bean extract containing a normal amount of hydroxyhydroquinone, the roasted coffee bean extract having a reduced hydroxyhydroquinone content has an improved facilitatory action on fat oxidation and energy metabolism. Accordingly, the roasted coffee bean extract containing chlorogenic acids or salts thereof and having the content of hydroxyhydroquinone of less than 0.1% by mass relative to the content of the chlorogenic acids can be used for enhancing fat oxidation or energy metabolism. The aforementioned use can be conducted in a human or non-human animal, or in a specimen derived therefrom, and the use may be either therapeutic or non-therapeutic.

Hence, in one aspect, the present invention provides a fat oxidation or energy metabolism enhancer comprising, as an active ingredient, a roasted coffee bean extract comprising chlorogenic acids or salts thereof and having the content of hydroxyhydroquinone of less than 0.1% by mass relative to the content of the chlorogenic acids.

In another aspect, the present invention provides a roasted coffee bean extract for use in enhancement of fat oxidation or energy metabolism, comprising chlorogenic acids or salts thereof and having the content of hydroxyhydroquinone of less than 0.1% by mass relative to the content of the chlorogenic acids.

Also, in another aspect, the present invention relates to use of a roasted coffee bean extract for the production of a fat oxidation or energy metabolism enhancer, comprising chlorogenic acids or salts thereof and having the content of hydroxyhydroquinone of less than 0.1% by mass relative to the content of the chlorogenic acids.

Also, in another aspect, the present invention provides a method for producing a fat oxidation or energy metabolism enhancer, comprising using a roasted coffee bean extract comprising chlorogenic acids or salts thereof as an active ingredient and having the content of hydroxyhydroquinone of less than 0.1% by mass relative to the content of the chlorogenic acids.

According to one embodiment, the fat oxidation or energy metabolism enhancer substantially consists of the aforementioned roasted coffee bean extract.

In a preferred aspect of the present invention, the content of the aforementioned chlorogenic acids in the aforementioned roasted coffee bean extract is 2 to 20% by mass relative to the total solid mass of the extract.

In another preferred aspect of the present invention, the content of the aforementioned hydroxyhydroquinone is 0.03% by mass or less relative to the content of the aforementioned chlorogenic acids.

In another preferred aspect of the present invention, the aforementioned fat oxidation and energy metabolism refer to postprandial fat oxidation and energy metabolism, respectively. Here, the term "postprandial" refers to time elapsed until most of the ingested dietary carbohydrate is absorbed, and it refers to the time from right after (0 minute) meal ingestion to six hours later, preferably five hours later, more preferably four hours later, and even more preferably three hours later (Diabete Care, 2001, 24 (4): 775 to 778).

In another preferred aspect of the present invention, the aforementioned roasted coffee bean extract, fat oxidation enhancer, or energy metabolism enhancer is used in an amount of 100 to 3000 mg in terms of the daily dose of chlorogenic acids for an adult.

In another preferred aspect of the present invention, the aforementioned roasted coffee bean extract is prepared by removing hydroxyhydroquinone from a roasted coffee bean extract.

In another preferred aspect of the present invention, the aforementioned roasted coffee bean extract is prepared by adding an adsorbent to a roasted coffee bean liquid extract or an aqueous solution of a dried roasted coffee bean extract, stirring the resulting solution at 0 to 100°C for 10 minutes to five hours, and then removing the adsorbent. The aforementioned adsorbent is preferably activated carbon.

The aforementioned roasted coffee bean extract can be used as an active ingredient of a pharmaceutical, a quasi-drug, a cosmetic, a food, a drink, a feed, and the like for enhancing fat oxidation or energy metabolism, or for the production of the products. The pharmaceutical, quasi-drug, cosmetic, food, drink, feed, and the like can be produced for or used in a human or non-human animal. Alternatively, the aforementioned roasted coffee bean extract can be blended, as a raw material, in a pharmaceutical, a quasi-drug, a cosmetic, a food, a drink, a feed, and the like for enhancing fat oxidation or energy metabolism.

The aforementioned pharmaceutical or quasi-drug contains the aforementioned roasted coffee bean extract as an active ingredient. The pharmaceutical or quasi-drug can be administered in any dosage form. The dosage form may be either in an oral dosage form or in a parenteral dosage form. Examples of the oral dosage form include a solid dosage form such as a tablet, a coated tablet, a granule, a powder, and a capsule as well as a liquid dosage form such as an elixirol, a syrup, and a suspension. Examples of the parenteral dosage form include an injection, an infusion solution, a transdermal dosage form, a transmucosal dosage form, a nasal dosage form, an enteral dosage form, an inhalation dosage form, a suppository, a bolus dosage form, an external preparation, and a patch.

The aforementioned pharmaceutical and quasi drug can contain the aforementioned roasted coffee bean extract alone or in combination with a pharmaceutically acceptable carrier. Examples of the carrier include an excipient, a coating agent, a binder, an expander, a disintegrant, a surfactant, a lubricant, a diluent, a dispersant, a buffer, an osmotic pressure adjuster, a pH adjuster, an emulsifier, a preservative, a stabilizer, an antioxidant, a colorant, an ultraviolet ray absorber, a humectant, a thickener, an activity enhancer, an anti-inflammatory agent, a germicide, a fragrance, a flavor, and an odor corrective. Also, as long as the facilitatory action of the aforementioned roasted coffee bean extract on fat oxidation and energy metabolism is not lost, the pharmaceutical and quasi drug can contain other active ingredients and pharmacological ingredients.

The aforementioned pharmaceutical and quasi drug can be produced by a routine method from the aforementioned roasted coffee bean extract or by combining the extract with the aforementioned carriers and/or other active ingredients and pharmacological ingredients as needed. The content of the aforementioned roasted coffee bean extract (in terms of chlorogenic acids) in the pharmaceutical or quasi drug is preferably 2 to 20% by mass, more preferably 5 to 18% by mass, and even more preferably 7 to 15% by mass.

The aforementioned cosmetic contains the aforementioned roasted coffee bean extract as an active ingredient. The aforementioned cosmetic can contain the roasted coffee bean extract alone or in combination with a carrier acceptable as a cosmetic.

Examples of the carrier include an excipient, a coating agent, a binder, an expander, a disintegrant, a surfactant, a lubricant, a diluent, a dispersant, a buffer, an osmotic pressure adjuster, a pH adjuster, an emulsifier, a preservative, a stabilizer, an antioxidant, a colorant, an ultraviolet ray absorber, a humectant, a thickener, an activity enhancer, an anti-inflammatory agent, a germicide, a fragrance, a flavor, and an odor corrective. Also, as long as the facilitatory action of the aforementioned roasted coffee bean extract on fat oxidation and energy metabolism is not lost, the cosmetic can contain other active ingredients and cosmetic ingredients such as a humectant, a whitening agent, an ultraviolet ray protectant, a cellular stimulant, a cleanser, a keratolytic agent, a makeup component (such as a makeup base, a foundation, a face powder, a powder, a blusher, a lipstick, an eye makeup cosmetic, an eyebrow, and a mascara).

Examples of the form of the cosmetic include any form which can be used in cosmetics such as a cream, an emulsion, a lotion, a suspension, a foam, a gel, a powder, a pack, a sheet, a patch, a stick, and a cake. For example, the cosmetic can be in the form of a lotion, emulsion, cream, gel, and the like used for body slimming.

The aforementioned cosmetic can be produced by a routine method from the aforementioned roasted coffee bean extract or by combining the extract with the aforementioned carriers and/or other active ingredients and cosmetic ingredients as needed. The content of the roasted coffee bean extract (in terms of chlorogenic acids) in the cosmetic is preferably 2 to 20% by mass, more preferably 5 to 18% by mass, and even more preferably 7 to 15% by mass.

Specific examples of the carrier acceptable in the aforementioned pharmaceutical or cosmetic include a solid carrier such as lactose, kaolin, sucrose, crystalline cellulose, corn starch, talc, agar, pectin, stearic acid, magnesium stearate, lecithin, and sodium chloride, and a liquid carrier such as glycerin, peanut oil, polyvinyl pyrrolidone, olive oil, ethanol, benzyl alcohol, propylene glycol, and water.

The aforementioned food and drink contain the aforementioned roasted coffee bean extract as the active ingredient, and they can be prepared as functional foods and drinks such as beauty foods and drinks, foods and drinks with nutrient function claims, foods and drinks for the sick, and foods and drinks for specified health uses, which are based on the concept of fat oxidation- or energy metabolism-facilitatory function and labeled with the functional information as needed. The kind of the aforementioned food and drink is not particularly limited. Examples of the drink include a drink of any kind such as a fruit juice drink, a carbonated drink, a tea drink, a coffee drink, a milk drink, an alcoholic drink, and a soft drink. The food can be in any form such as solid, semi-solid, and liquid, or it can also be in the form of a tablet, a pill, a tablet, a capsule, a liquid, a syrup, a powder, a granule, and the like. Examples of the food include breads, noodles, pasta, jelly foods, various kinds of snacks, cakes, confectionery, ice creams, soups, milk products, frozen foods, instant foods, other processed foods, seasonings, and supplements.

The aforementioned food and drink may contain the aforementioned roasted coffee bean extract alone or in combination with other food materials and an additive such as a solvent, a softener, oil, an emulsifier, a preservative, a fragrance, a stabilizer, a colorant, an antioxidant, a humectant, and a thickener. When the food and drink are in the form of a drink, the content of the roasted coffee bean extract (in terms of chlorogenic acids) in the food and drink is normally 0.01 to 5% by mass, preferably 0.05 to 3% by mass, more preferably 0.1 to 2% by mass, even more preferably 0.1 to 1% by mass, and even more preferably 0.15 to 1% by mass. Also, when the food and drink are in the form of jellies, the content is normally 0.01 to 1% by mass, preferably 0.02 to 0.5% by mass, and more preferably 0.05 to 0.3% by mass. Also, when the food and drink are in the form of a coffee drink, the content is normally 0.01 to 1% by mass, preferably 0.05 to 1% by mass, and more preferably 0.1 to 1% by mass, and even more preferably 0.15 to 1% by mass.

When the aforementioned roasted coffee bean extract is used as the active ingredient of a feed, examples of the pet food or feed include a feed for livestock such as cows, pigs, chickens, sheep, and horses, a feed for small animals such as rabbits, rats, and mice, a feed for fish and shellfish such as tunas, eels, sea breams, yellowtails, and shrimps, and a pet food used for feeding dogs, cats, little birds, squirrels, and the like. When producing a feed, the aforementioned roasted coffee bean extract is used alone or additionally mixed with ordinarily used feed ingredients, for example, meats such as beef, pork, and mutton, protein, grains, rice brans, sake lees, sugars, vegetables, vitamins, and minerals as needed. Further, gelling agents, shape retaining agents, pH adjusters, seasonings, preservatives, nutrition supplements, and the like, all of which are ordinarily used in a feed, may be mixed as needed. The above ingredients can then be processed by an ordinary method to produce the feed. The content of the aforementioned roasted coffee bean extract (in terms of chlorogenic acids) in the feed is 0.01 to 5% by mass, preferably 0.05 to 3% by mass, more preferably 0.1 to 2% by mass, even more preferably 0.1 to 1% by mass, and even more preferably 0.15 to 1% by mass.

The aforementioned roasted coffee bean extract may also be contained in the raw material for the aforementioned food, drink, and feed. Further, it can be the active ingredient of the aforementioned food, drink, and feed. For example, the aforementioned roasted coffee bean extract may be contained in a concentrated coffee liquid, a freeze-dried or spray-dried instant coffee, and the like, all of which serve as the raw material for the aforementioned coffee drink.

Further, the present invention provides a method for enhancing fat oxidation or energy metabolism, comprising administering the aforementioned roasted coffee bean extract or fat oxidation or energy metabolism enhancer. In one aspect, the method may be a non-therapeutic method based on the concept of body slimming for cosmetic purposes by enhancing fat metabolism. In this method, an effective dose of the roasted coffee bean extract or the fat oxidation or energy metabolism enhancer according to the present invention is administered to or ingested by a subject in need of enhancing fat oxidation or energy metabolism. Examples of the subject of administration or ingestion include animal, preferably a human or non-human mammals, more preferably a human.

The subject of administration or ingestion is not particularly limited as long as the subject is a human or animal in need of enhancement of fat oxidation or energy metabolism. Preferred examples of the subject include a human or animal in which body slimming is desired for cosmetic purposes, and more preferred examples include a human. Alternatively, preferred examples of the subject include people suffering from obesity or metabolic syndrome or those who are likely to develop those diseases. As to the standard of obesity, in Japan, those having a BMI = 25 or higher, and in Europe and America, those having a BMI = 30 or higher are mainly determined to be obese. As to the diagnostic criteria for metabolic syndrome for Japanese, males having a waist circumference of 85 cm or more or females having a waist circumference of 90 cm or more who satisfy at least one item of the following three items, which are (1) having blood triglyceride of 150 mg/dl or more or HDL cholesterol of less than 40 mg/dl, (2) having hyperglycemia (fasting blood glucose of 110 mg/dl or more), and (3) having hypertension (130/85 mHg or more) are determined to be likely to develop metabolic syndrome, and those who satisfy two or more items are determined to have metabolic syndrome. Thus, those people are preferable as the subject of the present invention. In the U.S., those who satisfy three or more of the waist circumference (102 cm or more for males and 88 cm or more for females), high triglyceride, low HDL, hypertension, and high fasting blood glucose are determined to have metabolic syndrome and those who satisfy two or more are likely to develop metabolic syndrome; thus, those people are preferable as the subject of the present invention.

Alternatively, the subject of administration or ingestion may be a tissue, an organ, and a cell collected from a human or animal in which enhancement of fat oxidation or energy metabolism is desired, or a fraction of these materials. The above tissue, organ, cell, or fraction of these materials is preferably a nature-derived or biologically or biotechnologically modified tissue, organ or cell, or fraction of these materials.

The effective amount of administration or ingestion of the fat oxidation enhancer of the present invention is not particularly limited as long as the subject of administration or ingestion can obtain a facilitatory effect on fat oxidation or energy metabolism. The effective amount can vary according to the condition, body weight, sex, and age of the subject or other factors. However, in the case of oral administration, normally, the daily dose for an adult is preferably 100 to 3000 mg, more preferably 300 to 2000 mg, and even more preferably 300 to 1000 mg in terms of chlorogenic acids. Also, while the aforementioned enhancer may be administered in accordance with any dosing regimen, it is preferably administered once or in several divided doses daily.

[Examples]

Hereinbelow, the present invention will be described more specifically with reference to Examples.

Reference Example 1 Preparation of a roasted coffee bean extract

Into a column-type extractor having a metal mesh, 60 kg of a blend of medium ground Brazilian beans roasted to a degree of L14 and to a degree of L16.5 (1 : 1), which were measured by a colorimeter, were placed, through which ion-exchanged water of 98°C was passed, whereby 413 kg of a roasted coffee bean liquid extract was obtained. The liquid extract thus obtained was appropriately fractionated and the concentration was adjusted, whereby a roasted coffee bean liquid extract before treatment with activated carbon was prepared (corresponding to the placebo drink to be described later), which had the following analytical values.

Concentration of hydroxyhydroquinone (HHQ): 36.5 mg/kg
Concentration of chlorogenic acids: 1805 mg/kg
Concentration of caffeine: 385.9 mg/kg

Subsequently, the above extract was passed through a column filled with the activated carbon Shirasagi WH2C 42/80L SS (manufactured by Japan EnviroChemicals, Ltd.) (amount of activated carbon relative to coffee solids: 50%), whereby an activated carbon-treated roasted coffee bean liquid extract was obtained (corresponding to the study drink to be described later), which had the following analytical values.

Concentration of HHQ: 1.5 mg/kg

Concentration of chlorogenic acids: 1686 mg/kg
Concentration of caffeine: 357 mg/kg

The concentrations of HHQ, chlorogenic acids, and caffeine in the aforementioned liquid extract were analyzed as follows.

(1) Analytical method for hydroxyhydroquinone:

As the analytical instrument, the CoulArray system (model 5600A, manufactured by ESA, U.S.A), which is an HPLC-electrochemical detector (coulometric type), was used. The names and model numbers of the constituent units of the instrument are as follows.

Analytical cell: Model 5010, CoulArray Organizer, CoulArray Electronics Module and Software: Model 5600A, Solvent Delivery Module: Model 582, Gradient Mixer, Autosampler: Model 542, Pulse Damper, Degasser: Degasys Ultimate DU3003, Column Oven:505, Column: CAPCELL PAK C18 AQ, Inner diameter 4.6 mm x Length 250 mm, Particle diameter 5 $\mu$m (Shiseido, Co., Ltd.).

The analytical conditions are as follows.
Sample injection volume: 10 $\mu$L, Flow rate: 1.0 mL/min
Voltage applied to the electrochemical detector: 0 mV
Column oven preset temperature: 40°C
Eluent A: 0.1(W/V)% phosphoric acid, 0.1 mM 1-hydroxyethane-1,1-diphosphonic acid, and 5(V/V)% methanol solution, Eluent B: 0.1(W/V)% phosphoric acid, 0.1 mM 1-hydroxyethane-1,1-diphosphonic acid, 50(V/V)% methanol solution. For preparation of the eluents A and B: Distilled water for high-performance liquid chromatography (Kanto Chemical Co., Inc.), methanol for high-performance liquid chromatography (Kanto Chemical Co., Inc.), phosphoric acid (special grade, Wako Pure Chemical Industries, Ltd.), and 1-hydroxyethane-1,1-diphosphonic acid (60% aqueous solution, Tokyo Chemical Industry Co., Ltd.).
Concentration gradient conditions

| Time | Eluent A | Eluent B |
|---|---|---|
| 0.0 minute | 100% | 0% |
| 10.0 minute | 100% | 0% |
| 10.1 minute | 0% | 100% |
| 20.0 minute | 0% | 100% |
| 20.1 minute | 100% | 0% |
| 50.0 minute | 100% | 0% |

For analysis, 5 g of sample was precisely weighed and made up to 10 mL with 0.5(W/V)% phosphoric acid, 0.5 mM 1-hydroxyethane-1,1-diphosphonic acid, and 5(V/V)% methanol solution. The resulting solution was centrifuged and the resulting supernatant was used as the analytical sample. The supernatant thus obtained was passed through Bond Elut SCX (Amount of the solid phase packing: 500 mg, Reservoir capacity: 3 mL, GL Sciences Inc.). The flow-through solution was collected after discarding approximately 0.5 mL of the initial flow-through solution. The flow-through solution thus obtained was filtered through a membrane filter (GL chromatodisc 25A, Pore diameter 0.45 $\mu$m, GL Sciences Inc.) and then immediately analyzed.

In the analysis under the aforementioned conditions, the retention time of HHQ was 6.38 minutes. Using hydroxyhydroquinone (Wako Pure Chemical Industries, Ltd.) as the standard substance, the % by mass was calculated from the obtained value of the peak area.

(2) Analytical method for chlorogenic acids:

As the analytical instrument, HPLC was used. The model numbers of the constituent units of the instrument are as follows.

UV-VIS detector: L-2420 (Hitachi High-Technologies Corporation), Column oven: L-2300 (Hitachi High-Technologies Corporation), Pump: L-2130 (Hitachi High-Technologies Corporation), Autosampler: L-2200 (Hitachi High-Technologies Corporation), Column: Cadenza CD-C18, Inner diameter 4.6 mm x Length 150 mm, Particle diameter 3 $\mu$m (Imtakt Corporation).

The analytical conditions are as follows.
Sample injection volume: 10 $\mu$L, Flow rate: 1.0 mL/min

UV-VIS detector preset wavelength: 325 nm
Column oven preset temperature: 35°C
Eluent C: 0.05 M acetic acid, 0.1 mM 1-hydroxyethane-1,1-diphosphonic acid, 10 mM sodium acetate, and 5(V/V)% acetonitrile solution, Eluent D: acetonitrile.
Concentration gradient conditions

| Time | Eluent C | Eluent D |
|---|---|---|
| 0.0 minute | 100% | 0% |
| 10.0 minute | 100% | 0% |
| 15.0 minute | 95% | 5% |
| 20.0 minute | 95% | 5% |
| 22.0 minute | 92% | 8% |
| 50.0 minute | 92% | 8% |
| 52.0 minute | 10% | 90% |
| 60.0 minute | 10% | 90% |
| 60.1 minute | 100% | 0% |
| 70.0 minute | 100% | 0% |

For analysis, 1 g of sample was precisely weighed and made up to 10 mL with the eluent A. The resulting solution was filtered through a membrane filter (GL chromatodisc 25A, Pore diameter 0.45 µm, GL Sciences Inc.) and then analyzed.
The retention time of chlorogenic acids (unit: minute)

(A1) Monocaffeoylquinic acid: A total of three points at 5.3, 8.8, and 11.6
(A2) Ferulaquinic acid: A total of three points at 13.0, 19.9, and 21.0
(A3) Dicaffeoylquinic acid: A total of three points at 36.6, 37.4, and 44.2.

Using 5-caffeoylquinic acid as the standard substance, the % by mass was obtained from the values of the areas of the nine kinds of chlorogenic acids obtained here.

(3) Analytical method for caffeine:

For analysis, HPLC (Shimadzu Corporation) was used. The model numbers of the constituent units of the instrument are as follows.
Detector: SPD-M10A, Oven: CTO-10AC, Pump: LC-10AD, Autosampler: SIL-10AD, Column: Inertsil ODS-2 (inner diameter 4.6 mm × length 250 mm).
The analytical conditions are as follows.
Sample injection volume: 10 µL, Flow rate: 1.0 mL/min
Detection wavelength of UV absorption spectrometer: 270 nm
Eluent E: a solution of 3% acetonitrile in 0.05 M acetic acid, Eluent F: a solution of 100% acetonitrile in 0.05 M acetic acid.
Concentration gradient conditions

| Time | Eluent E | Eluent F |
|---|---|---|
| 0 minute | 100% | 0% |
| 20 minute | 80% | 20% |
| 35 minute | 80% | 20% |
| 45 minute | 0% | 100% |
| 60 minute | 0% | 100% |
| 70 minute | 100% | 0% |
| 120 minute | 100% | 0% |

Example 1 Effect of intake of a roasted coffee bean extract without HHQ on postprandial energy metabolism

(Material and method)

Placebo drinks and study drinks were used as the test drinks. All of the drinks were subjected to retort sterilization and the like according to a routine method, whereby 185 g of canned coffee drinks were prepared. As the placebo drinks, the roasted coffee bean liquid extracts before treatment with activated carbon described in Reference Example 1 were used, in which the HHQ content was 6.75 mg/185 g. Meanwhile, as the study drinks, the activated carbon-treated roasted coffee bean liquid extracts described in Reference Example 1 were used, in which the HHQ content was 0.27 mg/185 g.

It should be noted that the placebo drinks and study drinks contained the same level of chlorogenic acids (334/185 g and 312 mg/185 g, respectively) and caffeine (71.4/185 g and 66.0 mg/185 g, respectively) as ordinary coffee drinks.

Ten healthy males in their 20s to 40s were selected as the subjects, in whom the effect of the roasted coffee bean extract without HHQ on postprandial energy metabolism was examined. The study was conducted as a double blind-crossover design, in which one week of the ingestion period for each test drink and one week of washout period before ingestion of each drink were set. The subjects were instructed to carry an activity meter with them and dietary restrictions were imposed (intake of foods and drinks containing coffee and teas containing catechin was prohibited) from the beginning of washout.

After completion of the washout period, the subjects were fasted overnight, and then the energy metabolism was assessed by the respiratory gas analysis method. Respiratory gas analysis was performed before meal intake and 3.5 hours after intake of meal + test drinks (meal contents: calories 540 kcal, protein 23 g, fat 16 g, and carbohydrate 76 g) using the Mass Spectrometer for Respiratory Analysis & Bioprocess Monitoring (ARCO2000 manufactured by Arcosystem Inc.).

Subsequently, during one week of ingestion period, the subjects were instructed to ingest one bottle of either placebo drink or study drink a day. It is to be noted that the placebo drinks and study drinks were ingested during the daytime activity, such as during working hours, without spending more than one hour. Also, during the period of dietary restriction, which was initiated two days before respiratory gas analysis, the subjects were instructed to ingest the drinks at a predetermined time (at breakfast).

After completion of the ingestion period, respiratory gas analysis was performed by a method similar to the method described earlier. After completion of the respiratory gas analysis, a series of "one week of washout → respiratory gas analysis → one week of drink ingestion (crossover) → respiratory gas analysis" was repeated once again.

Based on the measurement values of the amount of oxygen consumed and the amount of carbon dioxide produced, the respiratory exchange ratio and the amount of fat oxidation were calculated from the following calculation formula (Jequier et al., Annu Rev Nutr, 187 to 208, 1987).

$$\text{Respiratory exchange ratio} = \text{Amount of carbon dioxide produced} / \text{Amount of oxygen consumed}$$

$$\text{Amount of fat oxidation} = 1.689 \times (\text{Amount of oxygen consumed} - \text{Amount of carbon dioxide produced}),$$
$$(\text{mg/minute})$$

Also, given that energy metabolism is affected by muscle and cardiopulmonary activity, it was possible that the changes in the amount of activity due to exercise and the like might affect the results of the respiratory exchange ratio and the amount of fat oxidation. In light of this, in order to take the effect of the amount of activity into consideration, the activity of each test subject during the drink ingestion period was measured by a portable activity meter, and from the amount of activity thus measured and the subject's body weight, the energy consumed by each test subject during the drink ingestion period was calculated.

From each calculated value, a mean value and standard error in each of the placebo drink group and the study drink group were obtained, and a significance test was performed between these groups. The significance test was performed by a paired t-test (two tailed test, the level of significance $p < 0.05$).

(Results)

After one week of drink ingestion, the body weights were 67.8 ± 4.9 (at the initiation of ingestion: 68.1 ± 4.8) and 67.9 ± 4.8 (at the initiation of ingestion: 68.2 ± 4.9) kg in the placebo drink group and the study drink group, respectively, revealing that there was no significant difference in the body weight between the placebo drink group and the study drink group.

The daily average amounts of activity (amount of energy consumed) during the ingestion period of each drink were 2242.3 ± 242.8 kcal and 2153.2 ± 244.4 kcal in the placebo drink group and study drink group, respectively, revealing that there was no significant difference in the amount of daily activity between these groups.

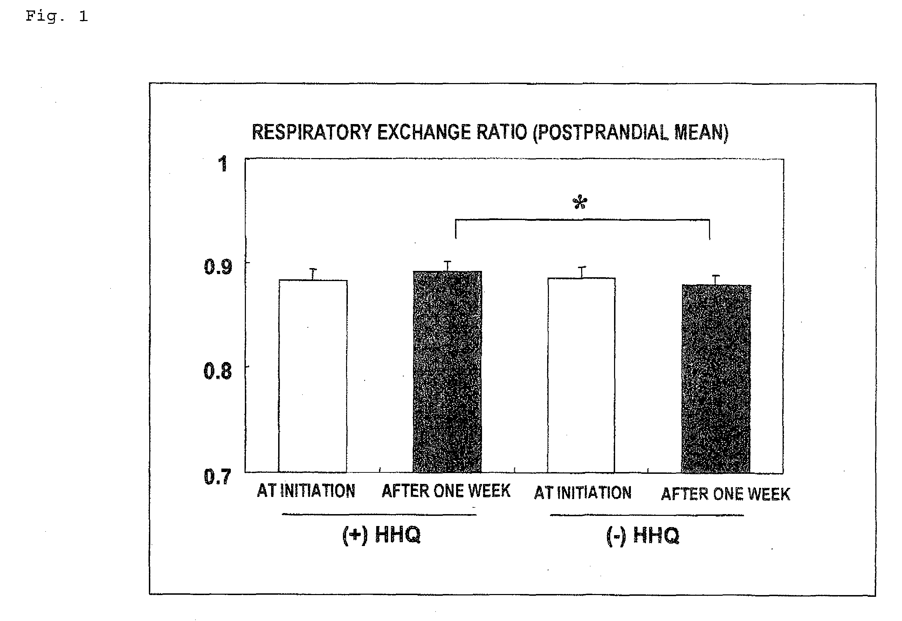

The mean postprandial values of the respiratory exchange ratio at the initiation of study and after one week of drink ingestion were shown in Figure 1. Compared to the placebo drink group ((+)HHQ), the study drink group ((-)HHQ) showed a significantly reduced value of the respiratory exchange ratio after one week of drink ingestion. The respiratory exchange ratio represents the oxidation ratio of carbohydrate and fat in energy consumption, indicating the smaller the value, the more the energy metabolism is shifted in favor of fat oxidation. Accordingly, the results obtained by this study showed that a roasted coffee bean extract without HHQ induced postprandial energy metabolism in favor of fat oxidation.

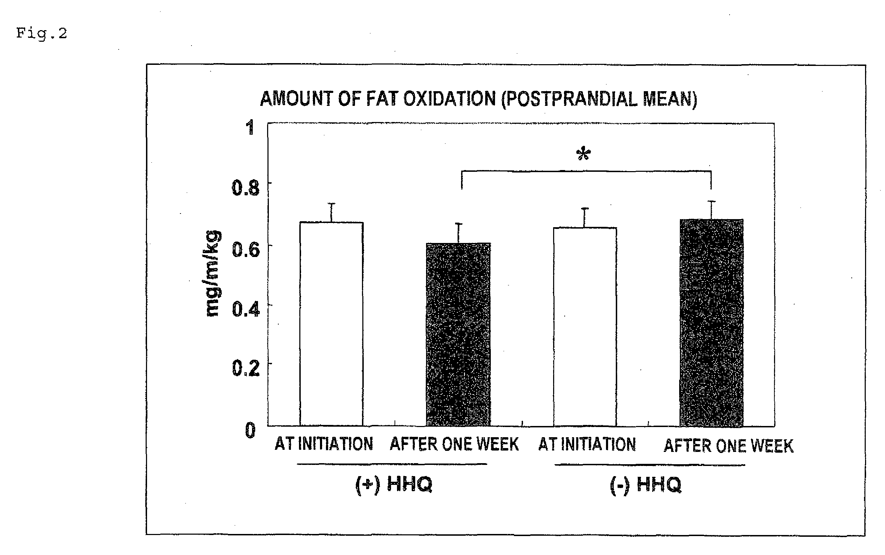

The mean values of the amount of postprandial fat oxidation were shown in Figure 2. After one week of drink ingestion, the study drink group ((-)HHQ) showed a significantly higher value of the amount of fat oxidation relative to the placebo drink group ((+)HHQ). The results showed that a roasted coffee bean extract without HHQ increased the amount of postprandial fat oxidation.

From the results of the respiratory exchange ratio and the amount of fat oxidation shown above, it was revealed that intake of a roasted coffee bean extract without HHQ induced postprandial energy metabolism in favor of fat oxidation, leading to enhancement of fat oxidation.

Example 2 Effect of intake of a roasted coffee bean extract without chlorogenic acid on postprandial energy metabolism

(Preparation of a roasted coffee bean extract)

A roasted coffee bean liquid extract (1) of a blend of medium ground Brazilian beans roasted to a degree of L14 and to a degree of L16.5 (1 : 1) was obtained. This liquid extract (1) was passed through a column filled with the activated carbon Shirasagi KL (manufactured by Japan EnviroChemicals, Ltd.) (amount of activated carbon relative to coffee solids: 50%). The liquid extract thus obtained was appropriately fractionated and the concentration was adjusted, whereby a roasted coffee bean liquid extract without chlorogenic acid (corresponding to the placebo drink to be described later) was prepared, which had the following analytical values.

Concentration of chlorogenic acids: 0 mg/kg
Concentration of HHQ: 0.03 mg/kg
Concentration of caffeine: 422 mg/kg

Also, the aforementioned roasted coffee bean liquid extract (1) was passed through a column filled with the activated carbon Shirasagi WH2C 42/80L SS (manufactured by Japan EnviroChemicals, Ltd.) (amount of activated carbon relative to coffee solids: 50%). The liquid extract thus obtained was appropriately fractionated and the concentration was adjusted, whereby a coffee bean liquid extract containing chlorogenic acid (corresponding to the study drink to be described later) was prepared, which had the following analytical values.

Concentration of chlorogenic acids: 1940 mg/kg
Concentration of HHQ: 0.57 mg/kg
Concentration of caffeine: 443 mg/kg

Seven healthy males in their 20s to 40s were selected as the subjects, in whom the effect of a roasted coffee bean extract without chlorogenic acid on postprandial energy metabolism was examined. Two drinks, namely a placebo drink (without chlorogenic acid) and a study drink (with chlorogenic acid), were used as the test drinks, and both were prepared as 185 g canned coffee drinks. The procedure of ingestion of the test drinks, respiratory gas analysis, and the like was performed by a method similar to that employed in Example 1, and respiratory gas analysis was performed before and after one week of ingestion.

(Results)

After one week of drink ingestion, the body weights were 67.2 ± 6.7 (at the initiation of ingestion: 67.1 ± 6.5) and 67.0 ± 6.8 (at the initiation of ingestion: 67.1 ± 6.9) kg in the placebo drink group and the study drink group, respectively, revealing that there was no significant difference in the body weight between the placebo drink group and the study drink group.

The daily average amounts of activity (amount of energy consumed) during the ingestion period of each drink were 2252.2 ± 241.1 kcal and 2230.9 ± 186.4 kcal in the placebo drink group and study drink group, respectively, revealing that there was no significant difference in the amount of daily activity between these groups.

The mean values of the amounts of oxygen consumed and fat oxidation at rest of each group after one week of drink ingestion were shown according to time in Table 1. After one week of drink ingestion, the study drink group showed a significantly higher value of the amount of oxygen consumed 30 to 150 minutes after meal intake relative to the placebo drink group. Also, as to the amount of fat oxidation, the study drink group showed a significantly higher value from 30 to 120 minutes after meal intake relative to the placebo drink group. Particularly, the study drink group showed an increase in the amount of fat oxidation in the early postprandial period.

From these results, it was revealed that intake of a roasted coffee bean extract containing chlorogenic acid and having a reduced amount of HHQ enhanced postprandial energy metabolism, particularly fat oxidation.

[Table 1]

The mean values of the amounts of oxygen consumed and fat oxidation according to time

| Time (min) | Amount of oxygen consumed (ml/kg par min) | | | Amount of fat oxidation (mg/kg par min) | | |
|---|---|---|---|---|---|---|
| | Chlorogenic acid (-) | Chlorogenic acid (+) | P | Chlorogenic acid (-) | Chlorogenic acid (+) | P |
| 30-60 | 3.23 ± 0.26 | 3.98 ± 0.16 | 0.026 | 0.681 ± 0.076 | 0.837 ± 0.093 | 0.066 |
| 30-90 | 3.33 ± 0.26 | 4.05 ± 0.15 | 0.031 | 0.665 ± 0.068 | 0.880 ± 0.083 | 0.021 |
| 30-120 | 3.36 ± 0.26 | 4.07 ± 0.14 | 0.043 | 0.662 ± 0.063 | 0.870 ± 0.088 | 0.048 |
| 30-150 | 3.35 ± 0.26 | 4.06 ± 0.13 | 0.049 | 0.677 ± 0.055 | 0.851 ± 0.092 | 0.066 |
| 30-180 | 3.30 ± 0.25 | 3.97 ± 0.12 | 0.053 | 0.668 ± 0.063 | 0.858 ± 0.084 | 0.053 |
| 30-210 | 3.26 ± 0.24 | 3.93 ± 0.12 | 0.055 | 0.695 ± 0.068 | 0.888 ± 0.092 | 0.060 |
| Means ± SEM (n = 7) | | | | | | |

## Claims

1. A roasted coffee bean extract for use in a method for preventing, ameliorating or reducing the risk of developing obesity or metabolic syndrome by enhancement of postprandial fat oxidation or postprandial energy metabolism, comprising chlorogenic acids or salts thereof and having a content of hydroxyhydroquinone of less than 0.1% by mass relative to a content of the chlorogenic acids.

2. The roasted coffee bean extract for use according to claim 1, wherein the content of the chlorogenic acids in the roasted coffee bean extract is 2 to 20% by mass relative to a total solid mass of the extract.

3. The roasted coffee bean extract for use according to claim 1 or 2, wherein the content of the hydroxyhydroquinone is 0.03% by mass or less relative to the content of the chlorogenic acids.

4. The roasted coffee bean extract for use according to any one of claims 1 to 3, wherein the roasted coffee bean extract is used in an amount of 100 to 3000 mg in terms of a daily dose of chlorogenic acids for an adult.

5. Non-therapeutic use of a roasted coffee bean extract for enhancement of postprandial fat oxidation, comprising chlorogenic acids or salts thereof and having a content of hydroxyhydroquinone of less than 0.1% by mass relative to a content of the chlorogenic acids.

6. Non-therapeutic use of a roasted coffee bean extract for enhancement of postprandial energy metabolism, comprising chlorogenic acids or salts thereof and having a content of hydroxyhydroquinone of less than 0.1% by mass relative to a content of the chlorogenic acids.

7. Non-therapeutic use according to claim 5 or 6, wherein the content of the chlorogenic acids in the roasted coffee bean extract is 2 to 20% by mass relative to a total solid mass of the extract.

**8.** Non-therapeutic use according to any one of claims 5 to 7, wherein the content of the hydroxyhydroquinone is 0.03% by mass or less relative to the content of the chlorogenic acids.

**9.** Non-therapeutic use according to claims 5-8, wherein the roasted coffee bean extract is used in an amount of 100 to 3000 mg in terms of a daily dose of chlorogenic acids for an adult.

**Patentansprüche**

**1.** Extrakt gerösteter Kaffeebohnen zur Verwendung in einem Verfahren zum Verhindern, Lindern oder Reduzieren des Risikos des Entwickelns von Fettleibigkeit oder dem metabolischen Syndrom durch Verbessern der postprandialen Fettoxidation oder des postprandialen Energiemetabolismus, das Chlorogensäuren oder Salze davon umfasst und einen Gehalt an Hydroxyhydrochinon von weniger als 0,1 Masse% in Bezug zum Gehalt der Chlorogensäuren aufweist.

**2.** Extrakt gerösteter Kaffeebohnen zur Verwendung gemäß Anspruch 1, worin der Gehalt der Chlorogensäuren im Extrakt der gerösteten Kaffeebohnen 2 bis 20 Masse%, relativ zur gesamten Feststoffmasse des Extrakts, ist.

**3.** Extrakt gerösteter Kaffeebohnen zur Verwendung gemäß Anspruch 1 oder 2, worin der Gehalt des Hydroxyhydrochinons 0,03 Masse% oder weniger, relativ zum Gehalt der Chlorogensäuren, ist.

**4.** Extrakt gerösteter Kaffeebohnen zur Verwendung gemäß irgendeinem der Ansprüche 1 bis 3, worin das Extrakt gerösteter Kaffeebohnen in einer Menge von 100 bis 3.000 mg in Bezug auf die Tagesdosis von Chlorogensäuren für einen Erwachsenen eingesetzt wird.

**5.** Nicht-therapeutische Verwendung eines Extrakts gerösteter Kaffeebohnen zur Verbesserung der postprandialen Fettoxidation, das Chlorogensäuren oder Salze davon umfasst und einen Gehalt von Hydroxyhydrochinon von weniger als 0,1 Masse%, relativ zum Gehalt der Chlorogensäuren, aufweist.

**6.** Nicht-therapeutische Verwendung eines Extrakts gerösteter Kaffeebohnen zum Verbessern des postprandialen Energiemetabolismus, das Chlorogensäuren oder Salze davon umfasst und einen Gehalt von Hydroxyhydrochinon von weniger als 0,1 Masse%, relativ zum Gehalt der Chlorogensäuren, aufweist.

**7.** Nicht-therapeutische Verwendung gemäß Anspruch 5 oder 6, worin der Gehalt der Chlorogensäuren im Extrakt gerösteter Kaffeebohnen 2 bis 20 Masse%, relativ zur gesamten Feststoffmasse des Extrakts, ist.

**8.** Nicht-therapeutische Verwendung gemäß Anspruch 5 bis 7, worin der Gehalt an Hydroxyhydrochinon 0,03 Masse% oder weniger, relativ zum Gehalt der Chlorogensäuren, ist.

**9.** Nicht-therapeutische Verwendung gemäß Anspruch 5 bis 8, worin das Extrakt gerösteter Kaffeebohnen in einer Menge von 100 bis 3.000 mg in Bezug auf eine Tagesdosis von Chlorgensäuren für einen Erwachsenen eingesetzt wird.

**Revendications**

**1.** Extrait de fèves de café torréfiées pour leur utilisation dans un procédé de prévention, d'amélioration ou de réduction du risque de développement d'une obésité ou d'un syndrome métabolique par l'amélioration de l'oxydation postprandiale des graisses ou du métabolisme énergétique postprandial, comprenant des acides chlorogéniques ou des sels de ceux-ci et ayant une teneur en hydroxyhydroquinone de moins de 0,1 % en poids par rapport à une teneur en acides chlorogéniques.

**2.** Extrait de fèves de café torréfiées pour leur utilisation selon la revendication 1, dans lequel la teneur en acides chlorogéniques dans l'extrait de fèves de café torréfiées est de 2 à 20 % en poids par rapport à un poids total de solides de l'extrait.

**3.** Extrait de fèves de café torréfiées pour leur utilisation selon la revendication 1 ou 2, dans lequel la teneur en hydroxyhydroquinone est de 0,03 % en poids ou moins par rapport à la teneur en acides chlorogéniques.

4. Extrait de fèves de café torréfiées pour leur utilisation selon l'une quelconque des revendications 1 à 3, l'extrait de fèves de café torréfiées étant utilisé en une quantité de 100 à 3000 mg en termes de dose quotidienne d'acides chlorogéniques pour un adulte.

5. Utilisation non thérapeutique d'un extrait de fèves de café torréfiées pour l'amélioration de l'oxydation postprandiale des graisses, comprenant des acides chlorogéniques ou des sels de ceux-ci et ayant une teneur en hydroxyhydroquinone de moins de 0,1 % en poids par rapport à une teneur en acides chlorogéniques.

6. Utilisation non thérapeutique d'un extrait de fèves de café torréfiées pour l'amélioration du métabolisme énergétique postprandial, comprenant des acides chlorogéniques ou des sels de ceux-ci et ayant une teneur en hydroxyhydroquinone de moins de 0,1 % en poids par rapport à une teneur en acides chlorogéniques.

7. Utilisation non thérapeutique selon la revendication 5 ou 6, dans laquelle la teneur en acides chlorogéniques dans l'extrait de fèves de café torréfiées est de 2 à 20 % en poids par rapport à un poids total de solides de l'extrait.

8. Utilisation non thérapeutique selon l'une quelconque des revendications 5 à 7, dans laquelle la teneur en hydroxyhydroquinone est de 0,03 % en poids ou moins par rapport à la teneur en acides chlorogéniques.

9. Utilisation non thérapeutique selon les revendications 5 à 8, dans laquelle l'extrait de fèves de café torréfiées est utilisé en une quantité de 100 à 3000 mg en termes de dose quotidienne d'acides chlorogéniques pour un adulte.

Fig. 1

RESPIRATORY EXCHANGE RATIO (POSTPRANDIAL MEAN)

EP 2 617 429 B1

Fig.2

AMOUNT OF FAT OXIDATION (POSTPRANDIAL MEAN)

EP 2 617 429 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003034636 A
- JP 2006271392 A
- JP 2008043238 A
- EP 2119368 A
- JP 2006087306 A
- JP 2006117631 A

**Non-patent literature cited in the description**

- *IUBMB Life,* 2006, vol. 58 (1), 39-46
- *J. Int. Med. Res.,* 2007, vol. 35 (6), 900-908
- *Eur. J. Clin. Nutr.,* 1999, vol. 53 (11), 831
- **CHO et al.** *Food and Chemical Toxicology,* 2010, vol. 48 (3), 937-943
- *Diabete Care,* 2001, vol. 24 (4), 775-778
- **JEQUIER et al.** *Annu Rev Nutr,* 1987, 187-208